Europäisches Patentamt

European Patent Office

Office européen des brevets

(19)

(11) **EP 0 963 368 B1**

(12) **FASCICULE DE BREVET EUROPEEN**

(45) Date de publication et mention
de la délivrance du brevet:
**19.09.2001 Bulletin 2001/38**

(21) Numéro de dépôt: **97952106.9**

(22) Date de dépôt: **18.12.1997**

(51) Int Cl.[7]: **C07C 57/145**, C08K 5/57,
C07F 7/22

(86) Numéro de dépôt international:
**PCT/FR97/02352**

(87) Numéro de publication internationale:
**WO 98/30531 (16.07.1998 Gazette 1998/28)**

(54) **COMPOSITION A BASE DE MALEATES D'ORGANOETAIN UTILISABLE POUR STABILISER ET LUBRIFIER DES POLYMERES THERMOPLASTIQUES. PROCEDE D'OBTENTION DE LADITE COMPOSITION**

**ZUSAMMENSETZUNG AUF DER BASIS VON ORGANOZINNMALEATEN, DIE FÜR DIE STABILISIERUNG UND SCHMIERUNG VON THERMOPLASTISCHEN POLYMEREN VERWENDBAR IST, UND VERFAHREN ZUR HERSTELLUNG DIESER ZUSAMMENSETZUNG**

**COMPOSITION WITH BASE OF ORGANOTIN MALEATES USEFUL FOR STABILISING AND LUBRICATING THERMOPLASTIC POLYMERS, METHOD FOR OBTAINING SAID COMPOSITION**

(84) Etats contractants désignés:
**AT BE CH DE DK ES FI FR GB GR IE IT LI LU NL PT SE**

(30) Priorité: **09.01.1997 FR 9700147**

(43) Date de publication de la demande:
**15.12.1999 Bulletin 1999/50**

(73) Titulaires:
• **Atofina**
**92800 Puteaux (FR)**
• **ELF ATOCHEM NORTH AMERICA, INC.**
**Philadelphia, Pennsylvania 19103-3222 (US)**

(72) Inventeurs:
• **BERTELO, Chris**
**Scotch Plains, NJ 07076 (US)**

• **CUILLERET, Muriel**
**F-69004 Lyon (FR)**
• **GIROIS, Stéphane**
**Jeffersonville, PA 19403 (US)**
• **MOREL, Patrick**
**F-60130 Ecully (FR)**

(74) Mandataire: **Ohresser, François**
**Atofina**
**D.C.R.D./D.P.I.**
**4, Cours Michelet**
**La Défense 10**
**92091 Paris La Défense Cedex (FR)**

(56) Documents cités:
**EP-A- 0 000 746        EP-A- 0 446 174**
**GB-A- 1 270 922**

**Description**

La présente invention a pour objet une composition constituée par des maléates d'organoétain, ainsi que son procédé d'obtention.

L'invention concerne également les compositions de polymères thermoplastiques stabilisées et lubrifiées à l'aide de ladite composition à base de maléates d'organoétain. Plus particulièrement, l'invention concerne des compositions de polymères de chlorure de vinyle à fusion contrôlée, stabilisées, et lubrifiées à l'aide de ladite composition à base de maléates d'organoétain.

Un autre objet de l'invention concerne les articles rigides extrudés obtenus à partir des compositions de polymères du chlorure de vinyle à fusion contrôlée, stabilisées et lubrifiées à l'aide de ladite composition à base de maléates d'organoétain.

On connaît un certain nombre de maléates d'organoétain constituant de bons agents de thermostabilisation et de photostabilisation des polymères de chlorure de vinyle.

Ainsi, des compositions de maléates d'organoétain tels que le bis-(isooctylmaléate) de dibutylétain, l'oxyde de bis(isooctylmaléate) de dibutylétain sont décrites dans la littérature. L'utilisation de ces compositions en tant que stabilisants thermiques pour des composés organiques halogénés, notamment des polymères halogénés tels que le polychlorure de vinyle (PVC) et le polychlorure de vinyle chloré (CPVC), est également bien connue.

L'efficacité des stabilisants à base de maléate d'organoétain pour le PVC, notamment des articles destinés à une utilisation extérieure est bien connue.

Ainsi, dans l'article intitulé "Worldwide Weathering of Polyvinyl Chloride", par Emery Szabo et Robert Lally ; Polymer and Engineering Science, avril 1975, Vol. 15, n° 4, on présente les résultats d'études d'exposition climatique à long terme. Les compositions stabilisantes utilisées étaient des compositions de savons de baryum/cadmium, des compositions de maléates d'organoétain et des compositions de mercaptoacétates d'organoétain. On note dans les conclusions que le DBTM (maléate de dibutylétain) domme les meilleurs résultats en tant que stabilisant UV et que les mercaptoacétates d'organoétain donne les résultats les plus médiocres.

Bien que la supériorité des compositions de maléates d'organoétain soit depuis longtemps reconnue pour la stabilisation de formulations de PVC soumises au vieillissement climatique, ces compositions n'ont pas réussi à satisfaire pleinement les besoins du marché en raison de nombreux inconvénients.

Ainsi, le brevet britannique 787 930 explique de la page 1 ligne 65 à la page 2 ligne 3, que le maléate de dibutylétain est difficile à disperser, et génère en outre lors de la transformation du PVC une fraction volatile d'anhydride maléique provoquant des effets lacrymogènes et irritants sur les personnes le manipulant. Afin de surmonter ces défauts, il est proposé dans le GB 787 930 des compositions d'hémiesters maléates d'organoétain liquides, telles que le bis(monobutylmaléate) de dibutylétain. La présence du groupement alcool de l'ester a pour effet de diminuer la teneur en étain, entraînant une stabilisation thermique moins importante, mais n'élimine pas la fraction volatile générée lors du mécanisme de stabilisation, c'est-à-dire la formation de l'hémiester d'acide maléique par la réaction du bis (monoalkylmaléate) d'organoétain avec l'HCl gazeux généré pendant la mise en oeuvre du PVC.

Le brevet américain 3 296 289 décrit des compositions stabilisantes d'hémiester maléate de diorganoétain améliorées, dans la mesure où ces compositions, constituées par des bis(hémiesters maléate) de diorganoétain, sont solides à température ambiante. Une composition typique du brevet US 3 296 289 était le bis(cyclohexylmaléate) de dibutylétain, ayant un point de fusion de 71°C à 73°C comme décrit à l'exemple 1. Bien que les compositions d'hémiester maléate d'organoétain solides décrites dans US 3 296 289 permettent de résoudre certains problèmes de manipulation, elles n'éliminent pas pour autant les composés volatils irritants générés lorsque les compositions stabilisantes sont soumises aux conditions thermiques appliquées lors de la mise en oeuvre du PVC qui s'effectue aux environs de 200°C.

Le brevet US 3 555 060 concerne des composés organiques d'étain tels que notamment le bis(isooctylmaléate) de dibutylétain.

Ces composés sont des chélates complexes présentant une liaison $\equiv$ Sn— O—Sn $\equiv$ avec 2 atomes d'étain par molécule.

Ces produits sont toutefois des liquides colorés, visqueux et difficiles à mettre en oeuvre.

Un autre problème qui se pose par l'utilisation des maléates d'organoétain comme stabilisant thermique des polymères de chlorure de vinyle est la difficulté de mise en oeuvre de ces forumulations. Il est en effet bien connu, notamment lors de l'extrusion de PVC rigide, que le polymère fondu contenant lesdits stabilisants présente une forte tendance au collage sur les équipements de transformation.

Pour remédier à ce problème, on utilise des combinaisons parfois complexes de lubrifiants, et on essaie plus rarement de modifier la structure du stabilisant lui-même.

Ainsi, le brevet GB 1378851 propose l'addition d'un mélange de cire de paraffine et d'un polymère acrylique lubrifiant à une formulation PVC contenant du dibutyl diméthylmaléate d'étain, de manière à réduire le collage sur les équipements. L'amélioration ainsi obtenue est caractérisée par une plus grande facilité à décoller un film transformé sur un malaxeur à cylindre, mais reste toutefois insuffisante.

Cependant, l'addition importante voire excessive de lubrifiants aux formulations PVC peut présenter des inconvénients. Ainsi, une teneur trop importante en lubrifiant interne aura, entre autres conséquences, d'augmenter la plastification du PVC et donc de diminuer son point Vicat. Dans le cas de formulations rigides, ceci peut constituer un élément rédhibitoire à l'utilisation des maléates d'organoétain.

De la même manière, un excès de lubrifiant externe, destiné à limiter les problèmes de collage lors de l'extrusion, aura tendance à favoriser les phénomères d'exsudation et donc de dépôts sur les équipements.

Ainsi, malgré plus de trente années de recherches, les stabilisants thermiques du PVC à base de maléates d'organoétain continuent d'avoir une utilisation limitée bien qu'ils présentent d'excellentes propriétés stabilisantes, et une très bonne résistance au vieillissement climatique. Leurs propriétés ne peuvent être considérées comme entièrement satisfaisantes :

- soit parce que lors de la mise en oeuvre, ils se décomposent en produits volatiles irritants et lacrymogènes,
- soit parce qu'ils sont difficiles à mettre en oeuvre.

On a maintenant trouvé une composition de maléates d'organoétain qui peut être obtenue en faisant réagir, éventuellement en milieu solvant et/ou en présence d'eau, un mélange constitué par au moins un composant RA et au moins un composant $R^1A$ avec l'anhydride maléique ou l'acide maléique, puis en mettant en contact le milieu réactionnel ainsi obtenu avec au moins un oxyde de dialkylétain $(R^2)_2 Sn = O$, ou au moins un chlorure d'alkylétain $(R^2)_x SnCl_{4-x}$, étant donné que :

RA représente

- soit un alcool ROH dans lequel R représente un radical hydrocarboné aliphatique, linéaire ou ramifié, ayant un nombre d'atomes de carbone allant de 1 à 10 et, de préférence compris entre 5 et 8, ou un mélange d'alcools de masse moléculaire moyenne en poids $\overline{Mw}$ supérieure à 32 et au plus égale à 158,
- soit un époxyalcane $C_nH_{2n}O$ dans lequel n va de 1 à 10 ou un mélange d'époxyalcanes de masse moléculaire moyenne en poids $\overline{Mw}$ supérieure à 30 et au plus égale à 156 ;

$R^1A$ représente

- soit un alcool $R^1OH$ dans lequel $R^1$ représente un radical hydrocarboné aliphatique, linéaire ou ramifié, ayant un nombre d'atomes de carbone allant de 20 à 50, ou un mélange d'alcools primaires saturés de masse moléculaire moyenne en poids $\overline{Mw}$ allant de 298 à 718,
- soit un époxyalcane $C_pH_{2p}O$ dans lequel p va de 20 à 50 ou un mélange d'époxyalcanes de masse moléculaire moyenne en poids $\overline{Mw}$ allant de 296 à 716 ;

$R^2$ représente un radical hydrocarboné aliphatique, linéaire ou ramifié, ayant un nombre d'atomes de carbone allant de 1 à 12, et de préférence égal à 1,4 ou 8,

x est un nombre entier égal à 1 ou 2.

Le polymère thermoplastique dans lequel on peut incorporer la composition à base de maléates d'organoétain en vue d'en améliorer notamment la stabilité thermique et la stabilité à la lumière peut notamment consister en un ou plusieurs polymères d'addition choisis dans le groupe formé par les homopolymères du chlorure de vinyle qui peuvent être éventuellement surchlorés et les copolymères, éventuellement greffés, qui résultent de la copolymérisation du chlorure de vinyle avec un ou plusieurs comonomères éthyléniquement insaturés. A titre de comonomères pour la préparation de tels copolymères conviennent notamment les halogénures de vinylidène comme le chlorure ou le fluorure de vinylidène, les carboxylates de vinyle comme l'acétate de vinyle, le propionate de vinyle, le butyrate de vinyle, les acides acrylique et méthacrylique ainsi que les nitriles, amides et esters d'alkyle qui en dérivent, notamment acrylonitrile, acrylamide, méthacrylamide, méthacrylate de méthyle, acrylate de méthyle, acrylate de butyle, acrylate d'éthyle, acrylate d'éthyl-2 hexyle, les dérivés vinylaromatiques tels que styrène, vinylnaphtalène, les oléfines telles que bicyclo (2.2.1)hept-2-ène, bicyclo(2.2.1)hepta-2,5-diène, éthylène, propène, butène-1.

Parmi ces polymères, l'invention concerne tout particulièrement les homo- et copolymères du chlorure de vinyle, éventuellement surchlorés.

La composition à base de maléates d'organoétain selon la présente invention peut être préparée selon le mode préféré ci-après qui consiste à réaliser d'abord, en milieu solvant et en chauffant, un mélange homogène et liquide des composants RA et $R^1A$. Ensuite, au mélange obtenu, porté à une température au moins égale à 50°C et, de préférence, comprise entre 70°C et 100°C, on ajoute d'abord l'anhydride maléique, en continu ou par portion, en une durée qui peut aller de 15 minutes à environ 1 heure, puis ensuite un oxyde de dialkylétain $(R^2)_2Sn=O$ en continu ou par portion, en une durée au moins égale à 15 minutes, et, de préférence allant de 30 minutes à 90 minutes.

Le milieu réactionnel est ensuite maintenu à une température allant de 50°C à 120°C et, de préférence, comprise entre 75°C et 100°C, pendant une durée qui est au moins égale à 15 minutes et, de préférence comprise entre 30 minutes et 90 minutes.

L'eau formée au cours de la réaction ainsi que le solvant peuvent être éliminés par distillation sous pression réduite à une température allant de 70°C à 120°C et, de préférence comprise entre 80°C et 100°C.

Le solvant utilisé doit être inerte vis-à-vis des réactifs et des produits formés.

A titre d'illustration de solvants utilisables selon la présente invention on citera, le toluène, les xylènes, l'heptane, le THF.

Selon la présente invention, on opère sous agitation et de préférence sous bullage de gaz inerte tel que l'azote.

On opère généralement à pression atmosphérique ($10^5$Pa), mais on ne sortirait pas du cadre de l'invention si on opérait à une pression différente.

Dans l'éventualité où l'on utilise des époxyalcanes, on opérera en présence d'eau. Celle-ci peut être avantageusement introduite avant l'addition de l'anhydride maléique.

Selon la présente invention, on utilisera les composants RA et $R^1A$ ainsi que l'anhydride maléique dans un rapport molaire anhydride maléique/(RA + $R^1A$) au moins égal à 1,5 et, de préférence compris entre 2 et 2,5.

Selon la présente invention, le rapport des pourcentages molaires RA/$R^1A$ peut varier dans une large mesure. Ce rapport peut aller de 1/99 à 99/1 et, de préférence, compris entre 20/80 et 80/20.

A titre d'illustration de composants RA utilisables selon la présente invention, on citera les alcools ROH tels que le méthanol, l'éthanol, le n-propanol, le n-butanol, le n-pentanol, le n-hexanol, le 2-éthylhexanol, le n-octanol, le n-décanol ou un mélange d'au moins deux des alcools précités ; les 1,2-époxyalcanes $C_nH_{2n}O$ tels que le 1,2-époxybutane, le 1,2-époxyhexane, le 1,2-époxyoctane, le 1,2-époxydécane ou un mélange d'au moins 2 des époxyalcanes précités.

On utilisera de préférence le 2-éthylhexanol.

A titre d'illustration de composants $R^1A$ utilisables selon la présente invention, on citera les alcools $R^1OH$ tels que le 1-nonacosanol (alcool montanylique), le hentriacontanol, le n-triacontanol (alcool myricylique), les mélanges d'alcools primaires saturés ayant une masse moléculaire moyenne en poids $\overline{Mw}$ allant de 375 à 700, une polydispersité $\overline{Mw}/\overline{Mn}$ voisine de 1 ($\overline{Mn}$ représentant la masse moléculaire moyenne en nombre) et une distribution régulière des radicaux hydrocarbonés aliphatiques linéaires en fonction de la longueur des chaînes hydrocarbonées, les mélanges de 1,2-époxyalcanes ayant une masse moléculaire moyenne en poids $\overline{Mw}$ allant de 313 à 632.

On utilisera de préférence un mélange d'alcools primaires saturés ayant une masse $\overline{Mw}$ égale à 460 ou un mélange d'époxyalcanes ayant une masse $\overline{Mw}$ égale à 632.

Selon la présente invention, on utilisera, pour 1 mole d'anhydride maléique au moins 0,50 mole d'oxyde de dialkylétain $(R^2)_2Sn = O$ et, de préférence, une quantité allant de 0,75 mole à 0,85 mole.

A titre d'illustration d'oxydes de dialkylétain utilisables selon la présente invention, on citera l'oxyde de diméthylétain, l'oxyde de dibutylétain, l'oxyde de dioctylétain.

Parmi ces composés, on préfère utiliser tout particulièrement l'oxyde de dibutylétain.

On peut également utiliser lors de la préparation de la composition à base de maléates d'organoétain au moins un chlorure d'alkylétain $(R^2)_xSnCl_{4-x}$ à la place d'un oxyde de dialkylétain $(R^2)_2Sn=0$.

De préférence, on utilisera un mélange de trichlorure de monoalkylétain $R^2SnCl_3$ et de dichlorure de dialkylétain $(R^2)_2SnCl_2$ ; les radicaux $R^2$ du trichlorure et du dichlorure pouvant être identiques ou différents.

5 Dans ce cas, les chlorures formés lors de la préparation de la composition peuvent être neutralisés par un hydroxyde alcalin.

La composition à base de maléates d'organoétain selon la présente invention possède une teneur pondérale en étain au moins égale à 10 % et, de préférence, comprise entre 15 % et 25 %.

0 Dans le cas général où les compositions obtenues sont solides à température ambiante, celles-ci peuvent être isolées par les moyens connus de l'homme du métier tels que notamment par coulage du milieu réactionnel chaud et liquide sur une surface refroidie puis écaillage du produit solidifié.

5 On ne sortirait pas du cadre de l'invention si la composition de maléates d'organoétain était obtenue en préparant séparément deux compositions de maléates d'organoétain contenant respectivement des composants RA de bas poids moléculaires et des composants $R^1A$ de haut poids moléculaires, puis en les mélangeant ultérieurement.

Sur la composition obtenue, on peut déterminer la teneur en étain par analyse élémentaire. Le spectre infrarouge de la composition selon l'invention se caractérise par :

- une bande d'absorption aux environs de 1730 cm$^{-1}$, caractéristique des fonctions esters,
- une bande d'absoption aux environs de 1580 cm$^{-1}$, caractéristique des carboxylates d'étain,
- une bande d'absorption aux environs de 680 cm$^{-1}$ caractéristique des liaisons $\equiv Sn - O - Sn \equiv$.

La composition à base de malétes d'organoétain peut être obtenue selon une variante qui consiste à introduire après l'élimination de l'eau formée, et éventuellement du solvant, une quantité de costabilisant au moins égale à 10 % en poids et, de préférence, comprise entre 15 % et 30 %, par rapport aux réactifs mis en oeuvre (hors eau).

Cette addition effectuée, on maintient le milieu réactionnel sous agitation et chauffage jusqu'à obtention d'un mélange homogène.

A titre d'illustration de costabilisants utilisables selon la présente invention, on citera les zéolithes, les hydrotalcites, les sels d'acides gras de calcium et de zinc.

L'invention a également pour objet un procédé d'obtention de maléates d'organoétain caractérisé en ce que l'on fait réagir un mélange constitué par au moins un composant RA et au moins un composant $R^1A$ avec l'anhydride maléique, puis que l'on met en contact le milieu réactionnel ainsi obtenu avec au moins un oxyde de dialkylétain $(R^2)_2Sn = O$ ou avec au moins un chlorure d'alkylétain $(R^2)_xSnCl_{4-x}$.

RA, $R^1A$, $(R^2)_2Sn=O$ et $(R^2)_xSnCl_{4-x}$ ont la même signification que précédemment donnée. Les conditions et paramètres opératoires, les réactifs utilisés et leurs proportions sont tels que définis ci-avant.

L'invention concerne également les compositions comprenant le polymère thermoplastique et la composition à base de maléates d'organoétain tels que définis ci-avant.

La composition à base de maléates d'organoétain peut être utilisée en des quantités allant de 0,5 à 5 parties en poids, de préférence de 1 à 4 parties, pour 100 parties en poids de polymère thermoplastique.

De telles compositions peuvent renfermer en outre, et en fonction notamment des conditions de mise en oeuvre ou de transformation et/ou des applications auxquelles elles sont destinées, les additifs usuels tels que pigments, charges, lubrifiants, agents de mise en oeuvre, modifiants choc, antioxydants, plastifiants et agents gonflants.

La composition à base d'étain telle qu'obtenue selon la présente invention peut être incorporée, en même temps, ou avant les additifs mentionnés précédemment lorsqu'il en est fait usage.

Selon une modalité particulièrement recommandée, on effectue cette opération dans un mélangeur rapide et on introduit successivement le polymère thermoplastique, la composition à base de maléates d'organoétain, puis les additifs et les charges.

En règle générale, cette opération peut s'effectuer à température ambiante, l'opération elle-même pouvant provoquer une élévation de température jusqu'à 70°C, voire davantage.

L'action stabilisante de la composition à base de maléates d'organoétain telle qu'obtenue selon la présente invention peut être mise en évidence en effectuant différents tests évaluant la stabilité thermique dynamique et statique des compositions de polymères thermoplastiques la contenant.

Les compositions de polymères thermoplastiques, notamment de PVC, comprenant la composition à base de maléates d'organoétain telle qu'obtenue selon la présente invention peuvent être moulées par injection, calandrées puis thermoformées, extrudées, coextrudées en articles rigides tels que revêtement intérieurs pour le bâtiment, huisseries, profilés de fenêtre, feuilles et tubes. Les articles transformés peuvent être compacts ou expansés.

L'action stabilisante à la chaleur et la lumière de la composition à base d'organoétain peut être mise en évidence pour les articles transformés par une mesure des coordonnées trichromatiques L*, a*, b*, le temps de stabilité résiduelle par un test de Rouge Congo et en soumettant ces articles aux rayonnements UV.

La composition de maléates d'organoétain telle qu'obtenue selon la présente invention présente les avantages, outre de stabiliser thermiquement les compositions de polymères thermoplastiques la contenant, d'en contrôler la gélification et la lubrification.

Notamment la composition de maléates d'organoétain de la présente invention permet d'obtenir une fusion contrôlée des compositions de polymères thermoplastiques la contenant. Ceci étant obtenu en faisant varier la nature, le ratio et la quantité totale des différents réactifs et plus particulièrement des composants RA et $R^1A$.

On a constaté que les composants $R^1A$ de haut poids moléculaire apportent un effet lubrifiant aux compositions de polymères thermoplastiques, tandis que les composants RA de bas poids moléculaire permettent de promouvoir la gélification et de contrôler le temps de fusion. Ces caractéristiques peuvent être mises en évidence en étudiant la rhéologie des compositions de polymères thermoplastiques à l'aide d'un rhéomètre à couple de torsion ou, par la lecture des paramètres de transformation lors de la mise en oeuvre. On a ainsi constaté que l'effet lubrifiant apporté par la présence de composant $R^1A$ de haut poids moléculaire dans la composition de maléates d'organoétain permet de réduire de manière significative l'addition de lubrifiant dans les compositions de polymères thermoplastiques.

La composition de maléates d'organoétain telle qu'obtenue selon la présente invention présente également l'avantage de ne pas altérer le point Vicat des compositions de polymères thermoplastiques transformées.

De plus, lors de la transformation des compositions de polymères thermoplastiques contenant la dite compositon de maléates d'organoétain, et plus particulièrement en extrusion, on n'observe pas de dégagement de produits irritants.

On a également observé que l'utilisation des composants RA et $R^1A$ lors de la préparation de la composition de maléates d'organoétain selon la présente invention, permettait de réduire, voire de supprimer la formation de dépôts sur les équipements utilisés pour la transformation des compositions de polymères thermoplastiques contenant la dite composition de maléates d'organoétain.

Les exemples qui suivent illustrent l'invention.

## I Préparation des compositions de maléates d'organoétain conformes à l'invention

Les compositions de maléates d'organoétain ont été préparées en utilisant les réactifs suivants :

- UNILIN® 425 (commercialisé par la Société PETROLITE) : mélange d'alcools primaires saturés ayant une masse moléculaire moyenne en poids $\overline{Mw}$ égale à 460, une polydispersité $\overline{Mw}/\overline{Mn}$ égale à 1, un point de fusion de 91°C, un nombre d'hydroxyle égal à 105 mg KOH/g d'échantillon déterminé selon la norme ASTM D 222 et un nombre moyen d'atomes de carbone égal à 30;
- UNILIN® 550 (commercialisé par la Société PETROLITE) :
mélange d'alcools primaires saturés ayant une masse moléculaire moyenne en poids $\overline{Mw}$ égale à 550, une polydispersité $\overline{Mw}/\overline{Mn}$ égale à 1, un point de fusion de 99°C, un nombre d'hydroxyle égal à 83 mg KOH/g d'échantillon déterminé selon la norme ATSM D 222 et un nombre moyen d'atomes de carbone égal à 40 ;
- VIKOLOX® C30+ (commercialisé par la société ELF ATOCHEM NORTH AMERICA Inc.) : mélange de 1,2-époxyalcanes ayant une masse moléculaire en poids $\overline{Mw}$ égal à 632, présentant un point de fusion de 72,7°C et un nombre moyen d'atomes de carbone égal à 45.
- VALFOR®100 (commercialisé par la société The PQ Corporation) : Zeolithe A de type aluminosilicate de sodium hydraté ;
- 2-éthylhexanol,
- anhydride maléique,
- oxyde de dibutylétain

### EXEMPLE 1 :

### PREPARATION D'UNE COMPOSITION DE MALEATES D'ORGANOETAIN SELON L'INVENTION, EN MILIEU SOLVANT.

Dans un réacteur double enveloppe de 500 ml, et muni d'une agitation, d'une gaine thermométrique, d'un condenseur équipé d'un Dean-Stark et d'une arrivée de gaz inerte, on introduit:

- 100 ml d'heptane (solvant),
- 84,2 g (0,183 mole) d'Unilin® 425,
- 5,9 g (0,045 mole) de 2-éthylhexanol,

Soit un rapport des pourcentages molaires 2-éthylhexanol / Unilin® 425 de 20/80.

Le mélange est chauffé vers 85°C et agité de manière à obtenir un milieu réactionnel liquide et homogène. Sous courant d'azote, on introduit 53,6 g (0,546 mole) d'anhydride maléique, en quatre portions sur 30 minutes, provoquant un léger exotherme à 95°C. Lorsque la température se stabilise à nouveau vers 85°C, on ajoute 109,5 g (0,440 mole) d'oxyde de dibutylétain en cinq portions sur 1 heure. Le milieu réactionnel est maintenu sous agitation et sous courant d'azote pendant 1 heure à 85°C. On augmente la température de 5°C à 10°C avant d'appliquer une pression de $6,66.10^3$ Pa. afin de distiller l'heptane et l'eau de réaction (environ 2 g d'eau). Après 1 heure de distillation, le réacteur est remis sous pression atmosphérique. On stoppe le chauffage et l'agitation, et on coule le milieu réactionnel sur une plaque métallique refroidie de façon à le solidifier.

On obtient environ 248 g de la compositon soit un rendement pondéral voisin de 99 %. Le pourcentage pondéral d'étain de la composition ainsi obtenue est de 20,8 %. Le spectre infra-rouge présente une bande d'absorption à 1729 cm-1 correspondant aux fonctions esters, une bande d'absorption à 1579 cm-1 correspondant aux fonctions carboxylate d'étain -C(O)-O-Sn≡ et une bande d'absorption à 680 cm-1 caractéristique des liaisons ≡Sn-O-Sn≡.

### EXEMPLE 2 :

### PREPARATION D'UNE COMPOSITION DE MALEATES D'ORGANOETAIN SELON L'INVENTION, UTILISANT UNE ZEOLITHE COMME COSTABILISANT.

On opère comme dans l'exemple 1 en utilisant les mêmes réactifs mais selon des quantités différentes. Ainsi on introduit :

- 100 ml d'heptane (solvant),
- 79,7 g (0,173 mole) d'Unilin® 425,
- 10,4 g (0,079 mole) de 2-éthylhexanol,

Soit un rapport des pourcentages molaires 2-éthylhexanol/Unilin® 425 d'environ 30/70.

- 53,6 g (0,546 mole) d'anhydride maléique,
- 109,5 g (0,440 mole) d'oxyde de dibutylétain.

Après l'élimination de l'heptane et de l'eau de réaction par distillation sous pression réduite, on remet le réacteur sous pression atmosphérique et on introduit 64 g de Valfor® 100 en quatre portions sur 30 minutes, sous agitation et en maintenant la température à 85°C. Après homogénéisation du milieu réactionnel le produit est isolé comme dans l'exemple 1.

On obtient environ 315 g de la composition soit un rendement pondéral voisin de 100 %. Le pourcentage pondéral d'étain de la composition ainsi obtenue est de 16,6 %. Le spectre infra-rouge présente des bandes similaires à celles de la composition de l'exemple 1.

## EXEMPLE 3

**PREPARATION D'UNE COMPOSITION SELON L'INVENTION, UTILISANT UN MELANGE DE 1,2-EPOXYALCANES, EN PRESENCE D'EAU ET EN ABSENCE DE SOLVANT.**

On opère selon les conditions opératoires de l'exemple 1, dans un réacteur identique, avec les quantités de réactifs suivants :

- 25 g d'eau,
- 79,7 g (0,126 mole) de Vikolox® C30+,
- 10,4 g (0,079 mole) de 2-éthylhexanol,

Soit un rapport des pourcentages molaires 2-éthylhexanol/Vikolox® C30 + d'environ 40/60.

- 53,6 g (0,546 mole) d'anhydride maléique,
- 109,5 g (0,440 mole) d'oxyde de dibutylétain.

La distillation sous pression réduite s'effectue de façon similaire à l'exemple 1 et on recueille 22 g d'eau après 1 heure. On introduit alors 64 g de Vaifor® 100 dans le milieu réactionnel comme décrit dans l'exemple 2.

Le produit est recueilli comme dans les exemples 1 et 2. On obtient envrion 319 g de la compositon soit un rendement pondéral voisin de 100 %. Le pourcentage pondéral d'étain de la composition ainsi obtenue est de 16,4 %.

Le spectre infra-rouge présente une bande d'absorption à 1736 cm-1 correspondant aux fonctions esters, une bande d'absoption à 1581 cm-1 correspondant aux fonctions carboxylates d'étain -C(O)-O-Sn≡ et une bande d'absorption à 681 cm-1 caractéristique des liaisons ≡Sn-O-Sn≡.

## EXEMPLE 4 :

**PREPARATION D'UNE COMPOSITION SELON L'INVENTION, UTILISANT UN MELANGE DE 1,2-EPOXYALCANES, EN PRESENCE D'EAU ET EN ABSENCE DE SOLVANT.**

On opère comme dans l'exemple 3 en utilisant les mêmes réactifs mais selon des quantités différentes. Ainsi on introduit :

- 25 g d'eau,
- 74,6 g (0,118 mole) de VIKOLOX® C30+,
- 15,4 g (0,118 mole) de 2-éthylhexanol,

Soit un rapport des pourcentages molaires 2-éthylhexanol/Vikolox® C30+ de 50/50,

- 53,6 g (0,546 mole) d'anhydride maléique,
- 109,5 g (0,440 mole) d'oxyde de dibutyfétain.

La distillation sous pression réduite s'effectue de façon similaire à l'exemple 1 et on recueille 23 g d'eau après 1 heure. On introduit alors 64 g de Valfor® 100 dans le milieu réactionnel comme décrit dans l'exemple 2.

Le produit est recueilli comme dans les exemples 1, 2 et 3. On obtient environ 319 g de la composition soit un rendement pondéral voisin de 100 %. Le pourcentage pondéral d'étain de la composition ainsi obtenue est de 16,4 %. Le spectre infra-rouge présente des bandes d'absorption similiaires à celles de l'exemple 3.

**EXEMPLE 5 :**

**PREPARATION D'UNE COMPOSITION SELON L'INVENTION SIMILAIRE A L'EXEMPLE 2, AVEC VARIATION DU RAPPORT DES POURCENTAGES MOLAIRES 2-ETHYLHEXANOL/UNILIN® 425.**

On opère comme dans l'exemple 2 en utilisant les mêmes réactifs mais selon des quantités différentes. Ainsi, on introduit :

- 100 ml d'heptane (solvant),
- 87,3 g (0,190 mole) d'Unilin® 425,
- 2,8 g (0,022 mole) de 2-éthylhexanol,

Soit un rapport des pourcentages molaires 2-éthylhexanol/Unilin® 425 de 10/90,

- 53,6 g (0,546 mole) d'anhydride maléique,
- 109,5 g (0,440 mole) d'oxyde de dibutylétain.

On introduit, comme dans l'exemple 2, 64 g de Valfor® 100 après la distillation sous pression résuite de l'heptane et de l'eau formée.

On obtient environ 315 g de la composition soit un rendement pondéral voisin de 100 %. Le pourcentage pondéral d'étain de la composition ainsi obtenue est de 16,6 %. Le spectre infra-rouge présente des bandes d'absorption similaires à celles de la composition de l'exemple 2.

**EXEMPLE 6 :**

**PREPARATION D'UNE COMPOSITION SELON L'INVENTION SIMILAIRE A L'EXEMPLE 2, AVEC VARIATION DU RAPPORT DES POURCENTAGES MOLAIRES ETHYLHEXANOL/UNILIN® 425.**

On opère comme dans l'exemple 2 en utilisant les mêmes réactifs mais selon des quantités différentes. Ainsi, on introduit :

- 100 ml d'hexane (solvant),
- 70,2 g (0.153 mole) d'Unilin® 425,
- 19,9 g (0,153 mole) de 2-éthylhexanol,

Soit un rapport des pourcentages molaires 2-éthylhexanol/Unilin® 425 de 50/50,

- 53,6 g (0,546 mole) d'anhydride maléique,
- 109,5 g (0,440 mole) d'oxyde de dibutylétain.

On introduit, comme dans l'exemple 2, 64 g de Valfor® 100 après la distillation sous pression réduite de l'heptane et de l'eau formée.

On obtient environ 314 g de la composition soit un rendement pondéral voisin de 100 %. Le pourcentage pondéral d'étain de la composition ainsi obtenue est de 16,6 %. Le spectre infra-rouge présente des bandes d'absorption similaires à celles de la composition de l'exemple 2.

**EXEMPLE 7 :**

**PREPARATION D'UNE COMPOSITION SELON L'INVENTION, UTILISANT L'UNILIN ® 550 EN REMPLACEMENT DE L'UNILIN® 425.**

On opère comme dans l'exemple 2 en utilisant les réactifs selon les quantités suivantes :

- 100 ml d'heptane (solvant),
- 82,5 g (0,150 mole) d'Unilin® 550,
- 8,2 g (0,063 mole) de 2-éthylhexanol,

Soit un rapport des pourcentages molaires 2-éthylhexanol/Unilin® 550 de 30/70,

- 53,6 g (0,546 mole) d'anhydride maléique,
- 109,5 g (0,440 mole) d'oxyde de dibutylétain.

On introduit, comme dans l'exemple 2, 64 g de Valfor® 100 après la distillation sous pression réduite de l'heptane et de l'eau formée.

On obtient environ 315 g de la compostion soit un rendement pondéral voisin de 100 %. Le pourcentage pondéral d'étain de la composition ainsi obtenue est de 16,6 %. Le spectre infra-rouge présente des bandes d'absorption similaires à celles de la composition de l'exemple 2.

**II Préparation des compositions de PVC, ci-après désignées par formulations PVC, contenant les compositons de maléates d'organoétain précédemment préparées et évalutation des propriétés stabilisantes et rhéologiques desdites compositions de maléates d'organoétain.**

Ci-après, on donne les noms commerciaux, les natures, les fournisseurs et les fonctions des différentes matières utilisées pour la préparation des formulations de PVC.

- LACOVYL S110P, résine PVC de K Wert égal à 67, ELF ATOCHEM S.A.,
- DURASTRENGTH 300, polymère acrylique, CECA S.A., modifiant choc,
- STAVINOR CAPSE, stéarate de calcium, CECA S.A., lubrifiant,
- METABLEN P551, polymère acrylique, METABLEN COMPANY B.V., agent de mise en oeuvre,
- KRONOS S2220, oxyde de titane, KRONOS, pigment,
- HYDROCARB 95T, carbonate de calcium, OMYA S.A., charge
- AC 316, cire de polyéthylène oxydée, ALLIED SIGNAL, lubrifiant.

ON PREPARE LES FORMULATIONS **PVC** SELON LES CONDITIONS OPERATOIRES SUIVANTES (LES QUANTITES DES MATIERES UTILISEES SONT EXPRIMEES EN POIDS) :

On introduit dans un mélangeur rapide Henschel muni d'une double enveloppe, avec une vitesse d'agitation de 3 800 tours/minute :

- 100 parties de résine PVC (Lacovyl S 110 P).

On observe une élévation de température.

A 60°C, on introduit 3,5 parties d'une composition de maléates d'organoétain telle qu'obtenue selon la présente invention, puis à 65°C, on introduit 0,2 partie de cire de polyéthylène oxydée (AC 316).

A 80°C, on ajoute :

- 0,6 partie de stéarate de calcium (Stavinor CA PSE),
- 7,5 parties de modifiant choc (Durastrength 300) et,
- 1 partie d'agent de mise en oeuvre (Metablen P 551).

Puis à 85°C, on ajoute :

- 4 parties d'oxyde de titane (Kronos S2220),
- 5 parties de carbonate de calcium (Hydrocarb 95 T).

La formulation PVC est soumise à une agitation de 3800 tours/minute jusqu'à atteindre une température de 110°C, puis refroidie en réduisant l'agitation à 1500 tours/minute et en faisant circuler un courant d'eau froide dans la double enveloppe du mélangeur.

La formulation obtenue est recueillie lorsque la température atteint environ 45°C.

On évalue sur un rhéomètre HAAKE-RHEOCORD 90 à 185°C, à une vitesse de 45 tours/minute et avec une charge de 65 g deux formulations PVC telles que précédemment préparées.

Les résultats sont reportés dans le tableau 1.

Dans ce tableau 1, on désigne par :

- Formulation PVC 1, une formulation contenant la composition de maléates d'organoétain de l'exemple 3 ;
- Formulation PVC-2, une formulation contenant la composition de maléates d'organoétain de l'exemple 4 ;
- Temps de dégradation, le temps au bout duquel on observe une élévation importante du couple liée à la réticulation et donc à la dégradation complète du PVC.

| | Formulation PVC 1 | Formulation PVC 2 |
|---|---|---|
| Temps de fusion (min) | 2,00 | 1,33 |
| Couple à la fusion (N.m) | 18,0 | 21,5 |
| Couple à l'équilibre (N.m) | 17,0 | 20,0 |
| Temps de dégradation (min) | 28,0 | 28,0 |

**TABLEAU 1**

On met ainsi en évidence l'influence du rapport molaire entre les alcools ou époxydes de bas poids moléculaire et de haut poids moléculaire, sur le temps de fusion et la viscosité (exprimée par le couple) de la formulation PVC à l'état fondu.

En augmentant la quantité d'alcool à bas poids moléculaire (2-éthylhexanol) par rapport à la quantité d'1,2-époxyalcane à haut poids moléculaire (Vikolox® C30+), toutes choses étant égales par ailleurs, on diminue significativement le temps de fusion.

A l'inverse, l'augmentation de la quantité d'1,2-époxyalcane à haut poids moléculaire entraîne une diminution importante du couple, mettant en évidence l'effet lubrifiant apporté par les longues chaînes.

Les mêmes formulations ont été transformées sur une extrudeuse KRAUSS MAFFEI KMDL25 équipée d'une filière plate 30 x 3 mm. Les paramètres relevés lors de l'extrusion sont présentés dans le tableau 2 :

| | Formulation PVC 1 | Formulation PVC 2 |
|---|---|---|
| Profil de température (°C) | 130 - 180 - 190 - 200 | |
| Température masse (°C) | 190 | 191 |
| Vitesse moteur (tr/min) | 15 | 15 |
| Couple (%) | 24 | 50 |
| Pression masse en tête (bar) | 120 | 134 |
| Débit (kg/h) | 4,0 | 4,0 |

**TABLEAU 2**

Les évaluations suivantes ont été effectuées sur les bandes extrudées :

- Colorimétrie (mesure des coordonnées trichromatiques L*, a*, b* et calcul des indices de jaune (YI) et des indices de blanc (WI) selon la norme ASTM E313) à l'aide d'un spectrocolorimètre Minolta Chromameter CR200,
- Température de ramolissement ou point Vicat, mesurée à l'aide d'un HDT Vicat Tester AFS,
- Stabilité résiduelle exprimée en minutes, mesurée à l'aide d'un banc Rouge-Congo Liebish.

Les résultats sont présentés dans le tableau 3 :

| | Formulation PVC 1 | Formulation PVC 2 |
|---|---|---|
| L* | 94,3 | 94,0 |
| a* | -0,74 | -0,71 |
| b* | 2,50 | 2,37 |
| YI | 3,7 | 3,6 |
| WI | 73,0 | 73,1 |
| Point Vicat (°C) | 79,4 | 79,4 |
| Rouge Congo (min) | 44 | 40 |

**TABLEAU 3**

On met de nouveau en évidence l'influence du rapport des pourcentages molaires des alcools ou 1,2-époxyalcanes de bas poids moléculaires et de haut poids moléculaire sur la lubrification de la formulation PVC, comme le montrent les valeurs de couple et de pression masse obtenues (tableau 2).

Les colorations des deux formulations PVC extrudées témoignent de l'efficacité des compositions de maléates d'organoétain obtenues selon la présente invention, pour prévenir la dégradation thermique du PVC durant sa mise en oeuvre. On montre également que ces compositions de maléates d'organoétain permettent de conserver un niveau élevé de point Vicat, conforme aux exigences de l'application.

On réalise un test de stabilité thermique dynamique sur trois autres formulations PVC préparées comme les formulations PVC 1 et 2.

On désigne ci-après :

- Formulation PVC 3, une formulation contenant la composition de maléates d'organoétain de l'exemple 5 ;
- Formulation PVC 4, une formulation contenant la composition de maléates d'organoétain de l'exemple 2 ;
- Formulation PVC 5,une formulation contenant la composition de maléates d'organoétain de l'exemple 6.

Ce test de stabilité thermique dynamique permet de mettre en évidence l'effet stabilisant remarquable apporté par les compositions décrites, variant par le rapport molaire des espèces RA et $R^1A$ de bas et haut poids moléculaire.

150 g des formulations PVC 3, 4 et 5 sont évaluées à l'aide d'un malaxeur à cylindre COLLIN, dont les rouleaux sont portés à 200°C. Les vitesses de rotation des deux cylindres sont respectivement réglées à 20tr/min et 24 tr/min de manière à gélifier puis travailler la matière entre les cylindres en apportant un travail mécanique de friction. L'écartement entre les cylindres est réglé à 0,7 mm.

Des échantillons sont prélevés sur les cylindres à intervalle de temps régulier en notant leur coloration jusqu'à dégradation totale.

Les indices de jaune (YI, norme ASTM E313) mesurés sur chaque échantillon prélevé, ainsi que le temps de dégradation correspondant au noircissement total sont présentés dans le tableau 4.

| | Formulation PVC 3 | Formulation PVC 4 | Formulation PVC 5 |
|---|---|---|---|
| YI (2 min) | 5,7 | 5,4 | 4,8 |
| YI (4 min) | 8,4 | 7,4 | 7,3 |
| YI (6 min) | 10,0 | 8,9 | 9,1 |
| YI (8 min) | 11,8 | 10,0 | 10,2 |
| YI (10 min) | 13,4 | 11,5 | 11,9 |
| YI (12 min) | 15,2 | 13,0 | 12,7 |
| YI (15 min) | 18,5 | 15,7 | 15,3 |
| YI (20 min) | 27,3 | 23,4 | 21,3 |
| YI (25 min) | 33,9 | 32,3 | 30,5 |
| YI (30 min) | 38,6 | 37,0 | 36,1 |
| Dégradation totale (min) | 45 | 45 | 50 |

**TABLEAU 4**

**III EVALUATION DES PROPRIETES FINALES DE PROFILES RIGIDES EN PVC FORMES A PARTIR DE FORMULATIONS PVC CONTENANT LES COMPOSITIONS DE MALEATES D'ORGANOETAIN.**

Afin de mettre en évidence les propriétés finales des profilés rigides stabilisés thermiquement par les compositions de maléates d'organoétain décrites dans l'invention, on a extrudé, parallèlement à la formulation PVC 4 et à la formulation PVC 6 (formulation PVC contenant la composition de maléates d'organoétain de l'exemple 7), une formulation PVC rigide stabilisée à l'aide de sels de plomb. Ce système stabilisant, bien connu de l'homme du métier, représente une référence dont l'efficacité est prouvée par son utilisation extensive par les transformateurs de profilés rigides.

Pour cela, on prépare une formulation PVC 7 selon le mode opératoire utilisé pour préparer les formulations PVC 1 à 6 excepté que l'on remplace les 3,5 parties de composition de maléates d'organoétain par une combinaison de 3,5 parties de phosphite dibasique de plomb (2PbO, $PbHPO_3$,1/2$H_2O$) et de 0,5 partie de stéarate dibasique de plomb (2PbO, $Pb(OOCC_{17}H_{35})_2$).

Un test de stabilité thermique dynamique permet de mettre en évidence l'effet stabilisant remarquable apporté par les compositions décrites :

150 g des formulations PVC 4, 6 et 7 sont évaluées à l'aide d'un malaxeur à cylindre COLLIN selon le mode opératoire décrit précédemment (évaluation des formulations PVC 3, 4 et 5 - tableau 4-). Les indices de jaune (YI, norme ASTM E313) mesurés sur chaque échantillon prélevé sont présentés dans le tableau 5.

|  | Formulation PVC 4 | Formulation PVC 6 | Formulation PVC 7 (référence) |
|---|---|---|---|
| YI (2 min) | 5,1 | 5,6 | 5,4 |
| YI (4 min) | 7,5 | 8,6 | 7,7 |
| YI (6 min) | 9,3 | 10,8 | 8,7 |
| YI (8 min) | 10,7 | 12,2 | 9,2 |
| YI (10 min) | 12,0 | 13,3 | 9,6 |
| YI (15 min) | 15,0 | 17,0 | 11,0 |

**TABLEAU 5**

Les trois formulations PVC 4, 6 et 7 ont également été transformées sur une extrudeuse industrielle REIFENHAÜSER BT65 équipée d'une filière en T pour profilés fenêtres.

Les paramètres relevés lors de l'extrusion sont présentés dans le tableau 6.

|  | Formulation PVC 4 | Formulation PVC 6 | Formulation PVC 7 (référence) |
|---|---|---|---|
| Profil de température (°C) dans le fourreau  zone 1 | 180 | 180 | 170 |
| zone 2 | 180 | 185 | 175 |
| zone 3 | 180 | 180 | 170 |
| zone 4 | 180 | 180 | 175 |
| dans la filière  zone 8 | 185 | 185 | 185 |
| zone 9 | 190 | 190 | 190 |
| Température de vis (°C) | 140 | 140 | 140 |
| Vitesse des vis (tr/min) | 25 | 25 | 25 |
| Couverture des vis | oui | oui | oui |
| Température masse (°C) | 190 | 181 | 187 |
| Ampérage | 7,3 | 5,7 | 7,5 |
| Débit (kg/h) | 62,8 | 58,6 | 59,8 |

**TABLEAU 6**

Les paramètres relevés lors de l'extrusion montrent que le comportement de la formulation PVC 4 (contenant la composition de l'exemple 2 obtenue avec l'Unilin® 425) est très proche de celui de la formulation PVC 7 (contenant le système de stabilisation au plomb).

En revanche, la formulation PVC 6 (contenant la composition de l'exemple 7 obtenu avec l'Unilin® 550) a un comportement rhéologique significativement différent, mettant une nouvelle fois en évidence l'effet lubrifiant des alcools de haut poids moléculaire. Cette formulation nécessiterait une optimisation de la lubrification afin d'obtenir un comportement rhéologique similaire à celui de la formulation PVC 7 au plomb.

Les profilés extrudés à partir des formulations PVC 4 et 7 ont été évalués et les résultats sont présentés dans le tableau 7.

| | Formulation PVC 4 | Formulation PVC 7 (référence) |
|---|---|---|
| Coloration de profilés | | |
| L* | 93,8 | 94,8 |
| a* | -0,43 | -0,36 |
| b* | 2,85 | 2,26 |
| Brillance 60° | 24,6 | 15,7 |
| Stabilité résiduelle (min) (Rouge Congo) | 38 | 60 |
| Température de ramollissement (°C) (Point Vicat) | 79,7 | 80,3 |

**TABLEAU 7**

Les propriétés mécaniques des profilés ont été évaluées par la mesure de la résilience en choc Charpy selon la norme DIN53753, utilisée en Allemagne pour l'évaluation des profilés rigides pour menuiserie extérieure. Les résultats sont présentés dans le tableau 8.

| | Formulation PVC 4 | Formulation PVC 7 (référence) |
|---|---|---|
| Résilience <R> en kJ/m² | 60,5 | 62,6 |

**TABLEAU 8**

On démontre ainsi l'efficacité des compositions de maléates d'organoétain décrites dans l'invention en tant que stabilisant thermique lors de la mise en oeuvre sur équipements industriels de formulations PVC rigides opaques destinées à la fabrication de profilés extrudés.

Elles permettent en effet d'assurer une mise en oeuvre aisée des formulations comme en témoignent les paramètres d'extrusion de la formulation PVC 4, très similaires à ceux obtenus pour une formulation de référence stabilisée avec des sels de plomb, en particulier au regard de l'ampérage du moteur d'entraînement des vis et du débit de l'extrudeuse.

Les colorations des profilés obtenus avec la formulation PVC 4, proches de celles observées avec la formulation de référence au plomb, montrent l'efficacité des composés à base de maléates d'organoétain pour stabiliser thermiquement les formulations et réduire ainsi la dégradation thermique du PVC lors de sa mise en oeuvre.

On note enfin que la valeur de résilience obtenue en choc Charpy pour les profilés stabilisés avec la composition à base de maléates d'organoétain (formulation PVC 4) est supérieure à la valeur de 50 kJ/m$^2$ préconisée par la norme DIN 53753.

**Revendications**

1. Composition de maléates d'organoétain telle qu'obtenue par la réaction d'un mélange constitué par au moins un composant RA et au moins un composant $R^1A$ avec l'anhydride maléique dans un rapport molaire anhydride maléique/(RA+$R^1$A) au moins égal à 1,5, puis mise en contact du milieu réactionnel ainsi obtenu avec au moins un oxyde de dialkyl étain $(R^2)_2 Sn = O$ ou avec au moins un chlorure d'alkylétain $(R^2)_x SnCl_{4-x}$, étant donné que :

RA représente

-   soit un alcool ROH dans lequel R représente un radical hydrocarboné aliphatique, linéaire ou ramifié, ayant un nombre d'atomes de carbone allant de 1 à 10 et, de préférence compris entre 5 et 8, ou un mélange d'alcools de masse moléculaire moyenne en poids, $\overline{Mw}$ supérieure à 32 et au plus égale à 158,
-   soit un époxyalcane $C_nH_{2n}O$ dans lequel n va de 1 à 10 ou un mélange d'époxyalcanes de masse moléculaire moyenne en poids $\overline{Mw}$ supérieur à 30 et au plus égale à 156,

$R^1A$ représente

-   soit un alcool $R^1OH$ dans lequel $R^1$ représente un radical hydrocarboné aliphatique, linéaire ou ramifié, ayant un nombre d'atomes de carbone allant de 20 à 50 ou un mélange d'alcools primaires saturés de masse moléculaire moyenne en poids $\overline{Mw}$ allant de 298 à 718,
-   soit un époxyalcane $C_pH_{2p}O$ dans lequel p va de 20 à 50 ou un mélange d'époxyalcanes de masse moléculaire moyenne en poids $\overline{Mw}$ allant de 296 à 716 ;

$R^2$ représente un radical hydrocarboné aliphatique, linéaire ou ramifié, ayant un nombre d'atomes de carbone allant de 1 à 12, et de préférence égal à 1,4 ou 8,
x est un nombre entier égal à 1 ou 2.

2.  Composition selon la revendication 1, **caractérisée en ce que** la réaction a lieu en présence d'un solvant.

3.  Composition selon la revendication 2, **caractérisée en ce qu'**elle est préparée selon les étapes suivantes qui consistent à réaliser en milieu solvant et en chauffant un mélange des composants RA et $R^1A$, puis à ajouter au mélange ainsi obtenu, porté à une température au moins égale à 50°C et, de préférence, comprise entre 70°C et 100°C, l'anhydride maléique en une durée allant de 15 minutes à 1 heure, puis ensuite à ajouter un oxyde de dialkylétain $(R^2)_2 Sn = O$ en une durée allant de 15 minutes à 90 minutes ; à chauffer le milieu reactionnel ainsi obtenu à une température allant de 50°C à 120°C et, de préférence, comprise entre 75°C et 100°C pendant une durée allant de 15 minutes à 90 minutes et à éliminer l'eau formée ainsi que le solvant sous pression 5 réduite à une température allant de 70°C à 120°C et, de préférence, comprise entre 80°C et 100°C.

4.  Composition selon l'une des revendications 1 à 3, **caractérisée en ce que** le rapport molaire anhydride maléique/ $(RA + R^1A)$ est compris entre 2 et 2,5.

5.  Composition selon l'une des revendications 1 à 3, **caractérisée en ce que** le rapport des pourcentages molaires $RA/R^1A$ va de 1/99 à 99/1 et, de préférence, compris entre 20/80 et 80/20.

6.  Composition selon l'une des revendications 1 à 3, **caractérisée en ce que** l'on utilise pour 1 mole d'anhydride maléique, au moins 0,50 mole d'oxyde de dialkylétain $(R^2)_2Sn = O$ et, de préférence, une quantité allant de 0,75 mole à 0,85 mole.

7.  Composition selon l'une des revendications 1 à 5, **caractérisée en ce que** le composé RA est un alcool ROH.

8.  Composition selon la revendication 7, **caractérisée en ce que** l'alcool ROH est le 2-éthylhexanol.

9.  Composition selon l'une des revendications 1 à 5, **caractérisée en ce que** le composé $R^1A$ est un mélange d'alcools primaires saturés ayant une masse moléculaire moyenne en poids $\overline{Mw}$ allant de 375 à 700 et une polydispersité $\overline{Mw}/\overline{Mn}$ voisine de 1 et, de préférence un mélange d'alcools ayant une masse $\overline{Mw}$ égale à 460 ou 550.

10. Composition selon l'une des revendications 1 à 5, **caractérisée en ce que** le composé $R^1A$ est un mélange d'époxyalcanes $C_pH_{2p}O$ ayant une masse moléculaire moyenne en poids $\overline{Mw}$ allant de 313 à 632 et, de préférence un mélange d'époxyalcanes ayant une masse $\overline{Mw}$ égale à 632.

11. Composition selon l'une des revendications selon 1 à 3 et 6, **caractérisée en ce que** l'oxyde de dialkylétain est l'oxyde de dibutylétain.

12. Composition selon la revendication 1, **caractérisée en ce que** l'on utilise un mélange de trichlorure de monoalkylétain $R^2SnCl_3$ et de dichlorure de dialkylétain $(R^2)_2SnCl_2$.

**13.** Composition selon l'une des revendications 1 à 12,
**caractérisée en ce que** la réaction a lieu en présence d'eau.

**14.** Composition selon l'une des revendications 1 à 13, **caractérisée en ce que** l'on utilise en outre un costabilisant.

**15.** Composition selon la revendication 14 **caractérisée en ce que** le costabilisant est une zéolithe.

**16.** Procédé pour obtenir une composition de maléates d'organoetain, selon la revendication 1, **caractérisé en ce que** l'on fait réagir un mélange constitué par au moins un composant RA et au moins un composant $R^1A$ avec l'anhydride maléique, dans un rapport molaire anhydride moléique / (RA+$R^1A$) au moins égal à 1,5, puis que l'on met en contact le milieu réactionnel ainsi obtenu avec au moins un oxyde de dialkyl étain $(R^2)_2Sn = O$ ou avec au moins un chlorure d'alkylétain $(R^2)_x SnCl_{4-x}$, étant donné que :

RA représente

- soit un alcool ROH dans lequel R représente un radical hydrocarboné aliphatique, linéaire ou ramifié, ayant un nombre d'atomes de carbone allant de 1 à 10 et, de préférence compris entre 5 et 8, ou un mélange d'alcools ROH de masse moléculaire moyenne en poids $\overline{Mw}$ supérieure à 32 et, au plus égale à 158,
- soit un époxyalcane $C_nH_{2n}O$ dans lequel n va de 1 à 10 ou un mélange d'époxyalcanes de masse moléculaire moyenne en poids $\overline{Mw}$ supérieure à 30 et, au plus égale à 156 ;

$R^1A$ représente

- soit un alcool $R^1OH$ dans lequel $R^1$ représente un radical hydrocarboné aliphatique, linéaire ou ramifié, ayant un nombre d'atomes de carbone allant de 20 à 50 ou un mélange d'alcools primaires saturés de masse moléculaire moyenne en poids $\overline{Mw}$ allant de 298 à 718,
- soit un époxyalcane $C_pH_{2p}O$ dans lequel p va de 20 à 50 ou un mélange d'époxyalcanes de masse moléculaire moyenne en poids $\overline{Mw}$ allant de 296 à 716 ;

$R^2$ représente un radical hydrocarboné aliphatique, linéaire ou ramifié, ayant un nombre d'atomes de carbone allant de 1 à 12, et de préférence égal à 1,4 ou 8,
x est un nombre entier égal à 1 ou 2.

**17.** Procédé selon la revendication 16, **caractérisé en ce que** l'on opère en milieu solvant.

**18.** Procédé selon la revendication 17, **caractérisé en ce que** l'on réalise en milieu solvant et en chauffant, un mélange des composants RA et $R^1A$, puis que l'on ajoute au mélange ainsi obtenu, porté à une température au moins égale à 50°C et, de préférence, comprise entre 70°C et 100°C, l'anhydride maléique en une durée allant de 15 minutes à 1 heure, puis ensuite que ion ajoute un oxyde de dialkylétain $(R^2)_2 Sn=O$ en une durée allant de 15 minutes à 90 minutes ; que l'on chauffe le milieu réactionnel ainsi obtenu à une température allant de 50°C à 120°C et, de préférence, comprise entre 75°C et 100°C pendant une durée allant de 15 5 minutes à 90 minutes et que l'on élimine l'eau formée ainsi que le solvant sous presssion réduite à une température allant de 70°C à 120°C et, de préférence, comprise entre 80°C et 100°C.

**19.** Procédé selon l'une des revendications 16 à 18, **caractérisé en ce que** le rapport molaire anhydride maléique/ (RA + $R^1A$) est compris entre 2 et 2,5.

**20.** Procédé selon l'une des revendications 16 à 18, **caractérisé en ce que** le rapport des pourcentages molaires RA/ $R^1A$ va de 1/99 à 99/1 et, de préférence, compris entre 20/80 et 80/20.

**21.** Procédé selon l'une des revendications 16 à 18, **caractérisé en ce que** l'on utilise pour 1 mole d'anhydride maléique, au moins 0,50 mole d'oxyde de dialkylétain $(R^2)_2Sn = O$ et, de préférence, une quantité allant de 0,75 mole à 0,85 mole.

**22.** Procédé selon l'une des revendications 16 à 20, **caractérisé en ce que** le composé RA est un alcool ROH.

**23.** Procédé selon la revendication 22, **caractérisé en ce que** l'alcool ROH est le 2-éthylhexanol.

**24.** Procédé selon l'une des revendications 16 à 20, **caractérisé en ce que** le composé R$^1$A est un mélange d'alcools primaires saturés ayant une masse moléculaire moyenne en poids $\overline{\text{Mw}}$ allant de 375 à 700 et une polydispersité $\overline{\text{Mw}}/\overline{\text{Mn}}$ voisine de 1 et, de préférence un mélange d'alcools ayant une masse $\overline{\text{Mw}}$ égale à 460 ou 550.

**25.** Procédé selon l'une des revendications 16 à 20, **caractérisé en ce que** le composé R$^1$A est un mélange d'époxyalcanes C$_p$H$_{2p}$O ayant une masse moléculaire moyenne en poids $\overline{\text{Mw}}$ allant de 313 à 632 et, de préférence un mélange d'époxyalcanes ayant une masse $\overline{\text{Mw}}$ égale à 632.

**26.** Procédé selon l'une des revendications 16 à 18 et 21, **caractérisé en ce que** l'oxyde de dialkylétain est l'oxyde de dibutylétain.

**27.** Procédé selon la revendication 16, **caractérisé en ce que** l'on opère avec un mélange de trichlorure de monoalkylétain R$^2$SnCl$_3$ et de dichlorure de dialkylétain (R$^2$)$_2$SnCl$_2$.

**28.** Procédé selon l'une des revendications 16 à 27, **caractérisé en ce que** l'on opère en présence d'eau.

**29.** Procédé selon l'une des revendications 16 à 28, **caractérisé en ce que** l'on opère en présence d'un costabilisant.

**30.** Procédé selon la revendication 29, **caractérisé en ce que** le costabilisant est une zéolithe.

**31.** Composition de polymère thermoplastique stabilisée et lubrifiée renfermant une composition de maléates d'organoétain selon l'une des revendications 1 à 15.

**32.** Composition selon la revendication 31, **caractérisé en ce que** le polymère thermoplastique est un homo- ou copolymère du chlorure de vinyle.

**33.** Article rigide extrudé compact, **caractérisé en ce qu'**il est formé à partir d'une composition de polymère thermoplastique selon l'une des revendications 31 et 32.

**Patentansprüche**

**1.** Zusammensetzung von Organozinnmaleaten, die durch Umsetzen eines Gemischs, das aus mindestens einem Bestandteil RA und mindestens einem Bestandteil R$^1$A besteht, mit Maleinsäureanhydrid in einem Molverhältnis Maleinsäureanhydrid/(RA + R$^1$A) von mindestens 1,5 und anschließendes Inkontaktbringen des so erhaltenen Reaktionsmediums mit mindestens einem Dialkylzinnoxid (R$^2$)$_2$Sn=O oder mit mindestens einem Alkylzinnchlorid (R$^2$)$_x$SnCl$_{4-x}$ hergestellt wird, wobei bedeuten:

- RA

  - einen Alkohol ROH, worin R einen geradkettigen oder verzweigten aliphatischen Kohlenwasserstoffrest bedeutet, der 1 bis 10 Kohlenstoffatome, vorzugsweise 5 bis 8 Kohlenstoffatome, enthält, oder ein Gemisch von Alkoholen mit einem Gewichtsmittel des Molekulargewichts $\overline{\text{Mw}}$ von mehr als 32 und höchstens 158, oder
  - ein Epoxyalkan C$_n$H$_{2n}$O, worin n im Bereich von 1 bis 10 liegt, oder ein Gemisch von Epoxyalkanen mit einem Gewichtsmittel des Molekulargewichts Mw von mehr als 30 und höchstens 156,

- R$^1$A

  - einen Alkohol R$^1$OH, worin R$^1$ einen geradkettigen oder verzweigten aliphatischen Kohlenwasserstoffrest bedeutet, der 20 bis 50 Kohlenstoffatome enthält, oder ein Gemisch gesättigter primärer Alkohole mit einem Gewichtsmittel des Molekulargewichts $\overline{\text{Mw}}$ von 298 bis 718, oder
  - ein Epoxyalkan C$_p$H$_{2p}$O, worin p im Bereich von 20 bis 50 liegt, oder ein Gemisch von Epoxyalkanen mit einem Gewichtsmittel des Molekulargewichts $\overline{\text{Mw}}$ von 296 bis 716,

- R$^2$ einen geradkettigen oder verzweigten aliphatischen Kohlenwasserstoffrest, der 1 bis 12 Kohlenstoffatome und vorzugsweise 1, 4 oder 8 Kohlenstoffatome enthält,
- x die ganze Zahl 1 oder 2.

**2.** Zusammensetzung nach Anspruch 1, **dadurch gekennzeichnet, daß** die Umsetzung in Gegenwart eines Lösemittels stattfindet.

**3.** Zusammensetzung nach Anspruch 2, **dadurch gekennzeichnet, daß** sie nach den folgenden Verfahrensschritten hergestellt wird, die darin bestehen, in einem Lösemittelmedium und unter Erhitzen ein Gemisch der Bestandteile RA und $R^1A$ herzustellen, anschließend zu dem so erhaltenen Gemisch, das bei einer Temperatur von mindestens 50 °C und vorzugsweise im Bereich von 70 bis 100 °C gehalten wird, Maleinsäureanhydrid während eines Zeitraums von 15 min bis 1 h zuzugeben, dann ein Dialkylzinnoxid $(R^2)_2Sn=O$ während eines Zeitraums von 15 bis 90 min zuzugeben, das so erhaltene Reaktionsmedium während eines Zeitraums von 15 bis 90 min auf eine Temperatur von 50 bis 120 °C und vorzugsweise im Bereich von 75 bis 100 °C zu erwärmen und das dabei gebildete Wasser sowie das Lösemittel unter vermindertem Druck bei einer Temperatur von 70 bis 120 °C und vorzugsweise im Bereich von 80 bis 100 °C zu entfernen.

**4.** Zusammensetzung nach einem der Ansprüche 1 bis 3, **dadurch gekennzeichnet, daß** das Molverhältnis Maleinsäureanhydrid / (RA + $R^1A$) im Bereich von 2 bis 2,5 liegt.

**5.** Zusammensetzung nach einem der Ansprüche 1 bis 3, **dadurch gekennzeichnet, daß** das Verhältnis der in Mol-% angegebenen Mengen von RA und $R^1A$ im Bereich von 1/99 bis 99/1, vorzugsweise im Bereich von 20/80 bis 80/20, liegt.

**6.** Zusammensetzung nach einem der Ansprüche 1 bis 3, **dadurch gekennzeichnet, daß** pro Mol Maleinsäureanhydrid mindestens 0,50 mol Dialkylzinnoxid $(R^2)_2Sn=O$ und vorzugsweise 0,75 bis 0,85 mol Dialkylzinnoxid verwendet werden.

**7.** Zusammensetzung nach einem der Ansprüche 1 bis 5, **dadurch gekennzeichnet, daß** die Verbindung RA ein Alkohol ROH ist.

**8.** Zusammensetzung nach Anspruch 7, **dadurch gekennzeichnet, daß** es sich bei dem Alkohol ROH um 2-Ethylhexanol handelt.

**9.** Zusammensetzung nach einem der Ansprüche 1 bis 5, **dadurch gekennzeichnet, daß** es sich bei der Verbindung $R^1A$ um ein Gemisch gesättigter primärer Alkohole mit einem Gewichtsmittel des Molekulargewichts $\overline{Mw}$ im Bereich von 375 bis 700 und einem Polymolekularitätsindex $\overline{Mw}$ /$\overline{Mn}$ von etwa 1 und vorzugsweise ein Gemisch von Alkoholen mit einem Gewichtsmittel Mw von 460 oder 550 handelt.

**10.** Zusammensetzung nach einem der Ansprüche 1 bis 5, **dadurch gekennzeichnet, daß** es sich bei der Verbindung $R^1A$ um ein Gemisch von Epoxyalkanen $C_pH_{2p}O$ mit einem Gewichtsmittel des Molekulargewichts $\overline{Mw}$ im Bereich von 313 bis 632 und vorzugsweise ein Gemisch von Epoxyalkanen mit einem Gewichtsmittel $\overline{Mw}$ von 632 handelt.

**11.** Zusammensetzung nach einem der Ansprüche 1 bis 3 und 6, **dadurch gekennzeichnet, daß** es sich bei dem Dialkylzinnoxid um Dibutylzinnoxid handelt.

**12.** Zusammensetzung nach Anspruch 1, **dadurch gekennzeichnet, daß** ein Gemisch aus Monoalkylzinntrichlorid $R^2SnCl_3$ und Dialkylzinndichlorid $(R^2)_2SnCl_2$ verwendet wird.

**13.** Zusammensetzung nach einem der Ansprüche 1 bis 12, **dadurch gekennzeichnet, daß** die Umsetzung in Gegenwart von Wasser durchgeführt wird.

**14.** Zusammensetzung nach einem der Ansprüche 1 bis 13, **dadurch gekennzeichnet, daß** außerdem ein Costabilisierungsmittel verwendet wird.

**15.** Zusammensetzung nach Anspruch 14, **dadurch gekennzeichnet, daß** es sich bei dem Costabilisierungsmittel um einen Zeolith handelt.

**16.** Verfahren zur Herstellung einer Zusammensetzung von Organozinnmaleaten nach Anspruch 1, **dadurch gekennzeichnet, daß** ein Gemisch, das aus mindestens einem Bestandteil RA und mindestens einem Bestandteil $R^1A$ besteht, mit Maleinsäureanhydrid in einem Molverhältnis Maleinsäureanhydrid/(RA + $R^1A$) von mindestens 1,5 umgesetzt wird und anschließendes das so erhaltene Reaktionsmedium mit mindestens einem Dialkylzinnoxid

$(R^2)_2Sn=O$ oder mit mindestens einem Alkylzinnchlorid $(R^2)_xSnCl_{4-x}$ in Kontakt gebracht wird, wobei bedeuten:

- RA

  - einen Alkohol ROH, worin R einen geradkettigen oder verzweigten aliphatischen Kohlenwasserstoffrest bedeutet, der 1 bis 10 Kohlenstoffatome, vorzugsweise 5 bis 8 Kohlenstoffatome, enthält, oder ein Gemisch von Alkoholen mit einem Gewichtsmittel des Molekulargewichts $\overline{Mw}$ von mehr als 32 und höchstens 158, oder
  - ein Epoxyalkan $C_nH_{2n}O$, worin ri im Bereich von 1 bis 10 liegt, oder ein Gemisch von Epoxyalkanen mit einem Gewichtsmittel des Molekulargewichts $\overline{Mw}$ von mehr als 30 und höchstens 156,

- $R^1A$

  - einen Alkohol $R^1OH$, worin $R^1$ einen geradkettigen oder verzweigten aliphatischen Kohlenwasserstoffrest bedeutet, der 20 bis 50 Kohlenstoffatome enthält, oder ein Gemisch gesättigter primärer Alkohole mit einem Gewichtsmittel des Molekulargewichts $\overline{Mw}$ von 298 bis 718, oder
  - ein Epoxyalkan $C_pH_{2p}O$, worin p im Bereich von 20 bis 50 liegt, oder ein Gemisch von Epoxyalkanen mit einem Gewichtsmittel des Molekulargewichts $\overline{Mw}$ von 296 bis 716,

- $R^2$ einen geradkettigen oder verzweigten aliphatischen Kohlenwasserstoffrest, der 1 bis 12 Kohlenstoffatome und vorzugsweise 1, 4 oder 8 Kohlenstoffatome enthält,
- x die ganze Zahl 1 oder 2.

17. Verfahren nach Anspruch 16, **dadurch gekennzeichnet, daß** in einem Lösemittelmedium gearbeitet wird.

18. Verfahren nach Anspruch 17, **dadurch gekennzeichnet, daß** in einem Lösemittelmedium und unter Erhitzen ein Gemisch der Bestandteile RA und $R^1A$ hergestellt wird, anschließend zu dem so erhaltenen Gemisch, das bei einer Temperatur von mindestens 50 °C und vorzugsweise im Bereich von 70 bis 100 °C gehalten wird, Maleinsäureanhydrid während eines Zeitraums von 15 min bis 1 h zugegeben wird, dann ein Dialkylzinnoxid $(R^2)_2Sn=O$ während eines Zeitraums von 15 bis 90 min zugegeben wird, das so erhaltene Reaktionsmedium während eines Zeitraums von 15 bis 90 min auf eine Temperatur von 50 bis 120 °C und vorzugsweise im Bereich von 75 bis 100 °C erwärmt wird und das dabei gebildete Wasser sowie das Lösemittel unter vermindertem Druck bei einer Temperatur von 70 bis 120 °C und vorzugsweise im Bereich von 80 bis 100 °C entfernt wird.

19. Verfahren nach einem der Ansprüche 16 bis 18, **dadurch gekennzeichnet, daß** das Molverhältnis Maleinsäureanhydrid/(RA + $R^1$ A) im Bereich von 2 bis 2,5 liegt.

20. Verfahren nach einem der Ansprüche 16 bis 18, **dadurch gekennzeichnet, daß** das Verhältnis der in Mol-% angegebenen Mengen von RA und $R^1A$ im Bereich von 1/99 bis 99/1, vorzugsweise im Bereich von 20/80 bis 80/20, liegt.

21. Verfahren nach einem der Ansprüche 16 bis 18, **dadurch gekennzeichnet, daß** pro Mol Maleinsäureanhydrid mindestens 0,50 mol Dialkylzinnoxid $(R^2)_2Sn=O$ und vorzugsweise 0,75 bis 0,85 mol Dialkylzinnoxid verwendet werden.

22. Verfahren nach einem der Ansprüche 16 bis 20, **dadurch gekennzeichnet, daß** es sich bei der Verbindung RA um einen Alkohol ROH handelt.

23. Verfahren nach Anspruch 22, **dadurch gekennzeichnet, daß** es sich bei dem Alkohol ROH um 2-Ethylhexanol handelt.

24. Verfahren nach einem der Ansprüche 16 bis 20, **dadurch gekennzeichnet, daß** es sich bei der Verbindung $R^1A$ um ein Gemisch gesättigter primärer Alkohole mit einem Gewichtsmittel des Molekulargewichts $\overline{Mw}$ im Bereich von 375 bis 700 und einem Polymolekularitätsindex $\overline{Mw}/\overline{Mn}$ von etwa 1 und vorzugsweise ein Gemisch von Alkoholen mit einem Gewichtsmittel $\overline{Mw}$ von 460 oder 550 handelt.

25. Verfahren nach einem der Ansprüche 16 bis 20, **dadurch gekennzeichnet, daß** es sich bei der Verbindung $R^1A$ um ein Gemisch von Epoxyalkanen $C_pH_{2p}O$ mit einem Gewichtsmittel des Molekulargewichts $\overline{Mw}$ im Bereich von 313 bis 632 und vorzugsweise ein Gemisch von Epoxyalkanen mit einem Gewichtsmittel $\overline{Mw}$ von 632 handelt.

26. Verfahren nach einem der Ansprüche 16 bis 18 und 21, **dadurch gekennzeichnet, daß** es sich bei dem Dialkylzinnoxid um Dibutylzinnoxid handelt.

27. Verfahren nach Anspruch 16, **dadurch gekennzeichnet, daß** ein Gemisch aus Monoalkylzinntrichlorid $R^2SnCl_3$ und Dialkylzinndichlorid $(R^2)_2SnCl_2$ verwendet wird.

28. Verfahren nach einem der Ansprüche 16 bis 27, **dadurch gekennzeichnet, daß** in Gegenwart von Wasser gearbeitet wird.

29. Verfahren nach einem der Ansprüche 16 bis 28, **dadurch gekennzeichnet, daß** in Gegenwart eines Costabilisierungsmittels gearbeitet wird.

30. Verfahren nach Anspruch 29, **dadurch gekennzeichnet, daß** es sich bei dem Costabilisierungsmittel um einen Zeolith handelt.

31. Gleitmittel enthaltende, stabilisierte thermoplastische Polymerzusammensetzung, die eine Zusammensetzung von Organozinnmaleaten nach einem der Ansprüche 1 bis 15 enthält.

32. Zusammensetzung nach Anspruch 31, **dadurch gekennzeichnet, daß** es sich bei dem thermoplastischen Polymer um ein Homo- oder Copolymer von Vinylchlorid handelt.

33. Kompakter, extrudierter, steifer Gegenstand, **dadurch gekennzeichnet, daß** er aus einer thermoplastischen Polymerzusammensetzung nach einem der Ansprüche 31 und 32 hergestellt ist.

**Claims**

1. Composition containing organotin maleates as obtained by the reaction of a mixture composed of at least one component RA and at least one component $R^1A$ with maleic anhydride, in a maleic anhydride/(RA + $R^1A$) molar ratio of at least 1.5, and then bringing the reaction mixture thus obtained into contact with at least one dialkyltin oxide $(R^2)_2Sn=O$ or with at least one alkyltin chloride $(R^2)_xSnCl_{4-x}$, given that:

   RA represents

   - either an alcohol ROH in which R represents a linear or branched aliphatic hydrocarbon radical having a number of carbon atoms ranging from 1 to 10 and preferably of between 5 and 8 or a mixture of alcohols with a weight-average molecular mass $\overline{Mw}$ greater than 32 and not more than 158,
   - or an epoxyalkane $C_nH_{2n}O$ in which n ranges from 1 to 10 or a mixture of epoxyalkanes with a weight-average molecular mass $\overline{Mw}$ greater than 30 and not more than 156;

   $R^1A$ represents

   - either an alcohol $R^1OH$ in which $R^1$ represents a linear or branched aliphatic hydrocarbon radical having a number of carbon atoms ranging from 20 to 50 or a mixture of saturated primary alcohols with a weight-average molecular mass $\overline{Mw}$ ranging from 298 to 718,
   - or an epoxyalkane $C_pH_{2p}O$ in which p ranges from 20 to 50 or a mixture of epoxyalkanes with a weight-average molecular mass $\overline{Mw}$ ranging from 296 to 716;

   $R^2$ represents a linear or branched aliphatic hydrocarbon radical having a number of carbon atoms ranging from 1 to 12 and preferably equal to 1, 4 or 8,
   x is an integer equal to 1 or 2.

2. Composition according to Claim 1, **characterized in that** the reaction takes place in the presence of a solvent.

3. Composition according to Claim 2, **characterized in that** it is prepared according to the following stages, which consist in producing a mixture of the components RA and $R^1A$ in solvent medium while heating, in then adding to the mixture thus obtained, which has been brought to a temperature of at least 50°C and preferably of between 70°C and 100°C, maleic anhydride over a period of time ranging from 15 minutes to 1 hour, and in then subsequently adding a dialkyltin oxide $(R^2)_2Sn=O$ over a period of time ranging from 15 minutes to 90 minutes; in heating the reaction mixture thus obtained at a temperature ranging from 50°C to 120°C and preferably of between 75°C and 100°C for a period of time ranging from 15 minutes to 90 minutes and in removing the water formed and the solvent under reduced pressure at a temperature ranging from 70°C to 120°C and preferably of between 80°C and 100°C.

4. Composition according to one of Claims 1 to 3, **characterized in that** the maleic anhydride/(RA + R$^1$A) molar ratio is between 2 and 2.5.

5. Composition according to one of Claims 1 to 3, **characterized in that** the ratio of the molar percentages RA/R$^1$A ranges from 1/99 to 99/1 and preferably of between 20/80 and 80/20

6. Composition according to one of Claims 1 to 3, **characterized in that** at least 0.50 mol of dialkyltin oxide $(R^2)_2Sn=0$ and preferably an amount ranging from 0.75 mol to 0.85 mol is used per 1 mol of maleic anhydride.

7. Composition according to one of Claims 1 to 5, **characterized in that** the compound RA is an alcohol ROH.

8. Composition according to Claim 7, **characterized in that** the alcohol ROH is 2-ethylhexanol.

9. Composition according to one of Claims 1 to 5, **characterized in that** the compound R$^1$A is a mixture of saturated primary alcohols having a weight-average molecular mass $\overline{Mw}$ ranging from 375 to 700 and a polydispersity $\overline{Mw}$/$\overline{Mn}$ in the region of 1 and preferably a mixture of alcohols having a mass $\overline{Mw}$ of 460 or 550.

10. Composition according to one of Claims 1 to 5, **characterized in that** the compound R$^1$A is a mixture of epoxy-alkanes $C_pH_{2p}O$ having a weight-average molecular mass $\overline{Mw}$ ranging from 313 to 632 and preferably a mixture of epoxyalkanes having a mass $\overline{Mw}$ of 632.

11. Composition according to one of Claims according to 1 to 3 and 6, **characterized in that** the dialkyltin oxide is dibutyltin oxide.

12. Composition according to Claim 1, **characterized in that** a mixture of monoalkyltin trichloride $R^2SnCl_3$ and of dialkyltin dichloride $(R^2)_2SnCl_2$ is used.

13. Composition according to one of Claims 1 to 12, **characterized in that** the reaction takes place in the presence of water.

14. Composition according to one of Claims 1 to 13, **characterized in that** a costabilizer is additionally used.

15. Composition according to Claim 14, **characterized in that** the costabilizer is a zeolite.

16. Process for producing a composition containing organotin maleates according to Claim 1, **characterized in that** a mixture composed of at least one component RA and at least one component R$^1$A is reacted with maleic anhydride, in a maleic anhydride/ (RA + R$^1$A) molar ratio of at least 1.5, and that the reaction mixture thus obtained is then brought into contact with at least one dialkyltin oxide $(R^2)_2Sn=O$ or with at least one alkyltin chloride $(R^2)_xSnCl_{4-x}$, given that:

RA represents

- either an alcohol ROH in which R represents a linear or branched aliphatic hydrocarbon radical having a number of carbon atoms ranging from 1 to 10 and preferably of between 5 and 8 or a mixture of alcohols ROH with a weight-average molecular mass $\overline{Mw}$ greater than 32 and not more than 158,
- or an epoxyalkane $C_nH_{2n}O$ in which n ranges from 1 to 10 or a mixture of epoxyalkanes with a weight-average molecular mass $\overline{Mw}$ greater than 30 and not more than 156;

R$^1$A represents

- either an alcohol R$^1$OH in which R$^1$ represents a linear or branched aliphatic hydrocarbon radical having a number of carbon atoms ranging from 20 to 50 or a mixture of saturated primary alcohols with a weight-average molecular mass $\overline{Mw}$ ranging from 298 to 718,

- or an epoxyalkane $C_pH_{2p}O$ in which p ranges from 20 to 50 or a mixture of epoxyalkanes with a weight-average molecular mass $\overline{Mw}$ ranging from 296 to 716;

$R^2$ represents a linear or branched aliphatic hydrocarbon radical having a number of carbon atoms ranging from 1 to 12 and preferably equal to 1, 4 or 8,

x is an integer equal to 1 or 2

**17.** Process according to Claim 16, **characterized in that** the operation is carried out in solvent medium.

**18.** Process according to Claim 17, **characterized in that** a mixture of the components RA and $R^1A$ is prepared in solvent medium while heating, that maleic anhydride is then added over a period of time ranging from 15 minutes to 1 hour to the mixture thus obtained, which has been brought to a temperature of at least 50°C and preferably of between 70°C and 100°C, and that a dialkyltin oxide $(R^2)_2Sn=O$ is then subsequently added over a period of time ranging from 15 minutes to 90 minutes; that the reaction mixture thus obtained is heated at a temperature ranging from 50°C to 120°C and preferably of between 75°C and 100°C for a period of time ranging from 15 minutes to 90 minutes and that the water formed and the solvent are removed under reduced pressure at a temperature ranging from 70°C to 120°C and preferably of between 80°C and 100°C.

**19.** Process according to one of Claims 16 to 18, **characterized in that** the maleic anhydride/ (RA + $R^1A$) molar ratio is between 2 and 2.5.

**20.** Process according to one of Claims 16 to 18, **characterized in that** the ratio of the molar percentages RA/$R^1A$ ranges from 1/99 to 99/1 and preferably of between 20/80 and 80/20.

**21.** Process according to one of Claims 16 to 18, **characterized in that** at least 0.50 mol of dialkyltin oxide $(R^2)_2Sn=0$ and preferably an amount ranging from 0.75 mol to 0.85 mol is used per 1 mol of maleic anhydride.

**22.** Process according to one of Claims 16 to 20, **characterized in that** the compound RA is an alcohol ROH.

**23.** Process according to Claim 22, **characterized in that** the alcohol ROH is 2-ethylhexanol.

**24.** Process according to one of Claims 16 to 20, **characterized in that** the compound $R^1A$ is a mixture of saturated primary alcohols having a weight-average molecular mass $\overline{Mw}$ ranging from 375 to 700 and a polydispersity $\overline{Mw}$/$\overline{Mn}$ in the region of 1 and preferably a mixture of alcohols having a mass $\overline{Mw}$ of 460 or 550.

**25.** Process according to one of Claims 16 to 20, **characterized in that** the compound $R^1A$ is a mixture of epoxyalkanes $C_pH_{2p}O$ having a weight-average molecular mass $\overline{Mw}$ ranging from 313 to 632 and preferably a mixture of epoxyalkanes having a mass $\overline{Mw}$ of 632.

**26.** Process according to one of Claims 16 to 18 and 21, **characterized in that** the dialkyltin oxide is dibutyltin oxide.

**27.** Process according to Claim 16, **characterized in that** the operation is carried out with a mixture of monoalkyltin trichloride $R^2SnCl_3$ and of dialkyltin dichloride $(R^2)_2SnCl_2$.

**28.** Process according to one of Claims 16 to 27, **characterized in that** the operation is carried out in the presence of water.

**29.** Process according to one of Claims 16 to 28, **characterized in that** the operation is carried out in the presence of a costabilizer.

**30.** Process according to Claim 29, **characterized in that** the costabilizer is a zeolite.

**31.** Stabilized and lubricated thermoplastic polymer composition including a composition containing organotin maleates according to one of Claims 1 to 15.

**32.** Composition according to Claim 31, **characterized in that** the thermoplastic polymer is a homo- or copolymer of vinyl chloride.

**33.** Compact extruded rigid article, **characterized in that** it is formed from a thermoplastic polymer composition according to either of Claims 31 and 32.